# EUROPEAN PATENT APPLICATION

(11) **EP 4 442 259 A1**
(43) Date of publication of application: **09.10.2024**
(21) Application number: 22901370.1
(22) Date of filing: 30.11.2022
(51) Int. Cl.: A61K 31/20, A61K 31/202, A61P 17/18, A61P 25/16, A61P 25/28, A61P 27/02, A61P 35/00, A61P 37/08, A61P 39/06

(54) **METHOD FOR ALLEVIATING OXIDATIVE STRESS**

(30) Priority: 03.12.2021 US 202163285669 P
(71) Applicant: Nissui Corporation, Tokyo 105-8676 (JP)
(72) Inventor: NAKA, Tadaomi, Hachioji-shi, Tokyo 192-0991 (JP); SEKI, Wakako, Hachioji-shi, Tokyo 192-0991 (JP); SATO, Seizo, Hachioji-shi, Tokyo 192-0991 (JP)
(74) Representative: Hoffmann Eitle
(86) International application number: PCT/JP2022/044201
(87) International publication number: WO 2023/100938

(57) **Abstract**

The present invention provides a method for treating or preventing an oxidative stress-related disorder, the method comprising administering an effective amount of a very-long-chain polyunsaturated fatty acid (VLC-PUFA), a pharmaceutically functional derivative thereof or a pharmaceutically acceptable salt thereof to a subject in need of the treatment or prevention.

## Description

### TECHNICAL FIELD

The present invention relates to a method for alleviating oxidative stress in a living organism which is caused by reactive oxygen species. The present invention also relates to a method for treating or preventing a disease or disorder caused by oxidative stress.

### BACKGROUND ART

For many organisms, oxygen is a substance that is absolutely necessary to live. On the other hand, reactive oxygen species (ROS) such as hydrogen peroxide and hydroxyl radicals which are generated by oxygen entrapped in a living organism are known to affect tissues and cells in the living organism. A living organism has functions of inhibiting generation of reactive oxygen species and eliminating the generated reactive oxygen species. These functions protect tissues and cells from oxidative stress, but if ROS are produced to the extent of exceeding the treatment capacity of the antioxidative functions, oxidative stress is induced. The oxidative stress is stress caused by an imbalance between reactive oxygen species present in a living organism, in particular, in cells and the ability of cells to neutralize the reactive oxygen species. The reactive oxygen species are characterized by superoxide anions that may damage nucleic acids, proteins and lipids of cells. The damage to nucleic acids such as deoxyribonucleic acid (DNA) may include damage to bases, and double-strand breaks. The damage to proteins may disrupt signaling and functions of cells. The reactive oxygen species-mediated damage to lipids may disorder cell membranes.

The oxidative stress is related to various diseases, and considered to be involved in the onset of, for example, eye diseases, neurological diseases, Down syndrome, cancer, cardiovascular diseases, respiratory diseases, lifestyle-related diseases, skin disorders, gastrointestinal diseases, liver diseases, kidney diseases, autoimmune diseases, otorhinolaryngologic diseases, sepsis, stress after organ transplantation, chronic fatigue syndrome and age-related diseases.

It is known that fish is rich in n-3 polyunsaturated fatty acids as unsaturated fatty acids. Many studies have been heretofore reported which show that the risk of having a cardiovascular disease decreases as the frequency to eat fish increases. In particular, as a pioneering study that has drawn attention to eicosapentaenoic acid (EPA), an n-3 polyunsaturated fatty acid, in prevention or treatment of a cardiovascular disease, an epidemiological study in Greenland is widely known. The epidemiological study has revealed that in the Inuit that are native people of Greenland, the number of people suffering from acute myocardial infarction is small, and the rate of death from ischemic cardiac disease is low. Further, their dietary habits have been examined, and the results have demonstrated that they have a dietary habit in which a lot of seal and fish are eaten, so that n-3 polyunsaturated fatty acids such as EPA and docosahexaenoic acid (DHA) are taken in abundance, whereas the intake of n-6 polyunsaturated fatty acids, contained in a large amount in terrestrial animals and plants is limited. Such a distinct constitution of fatty acids taken through diet has been reported to be a factor of the low rates of incidence of acute myocardial infarction and death from ischemic cardiac disease (Non Patent Literatures 1 to 3). EPA is used as a medical drug in treatment of arteriosclerosis obliterans and hyperlipidemia.

EPA has been reported to suppress oxidation of high-density lipoprotein (HDL) (Non Patent Literature 4). In addition, studies on antioxidative activity of polyunsaturated fatty acids have been reported (Patent Literatures 1 to 4 and Non Patent Literature 5). In recent years, there has been reports on the acquisition methods and activity of very-long-chain polyunsaturated fatty acids (VLC-PUFA, for example, n-3 VLC-PUFA) having a longer carbon chain over DHA and EPA, as well as their hydroxylated derivatives thereof known as elovanoid (Patent Literatures 5 to 7 and Non Patent Literature 6).

### CITATION LIST

### PATENT LITERATURE

PTL 1: Japanese Translation of PCT International Application Publication No. 2014-527029A
PTL 2: Japanese Patent Laid-Open No. 2016-138138A
PTL 3: Japanese Patent Laid-Open No. 2017-214305A
PTL 4: Japanese Translation of PCT International Application Publication No. 2013-509439A
PTL 5: Japanese Translation of PCT International Application Publication No. 2018-506584
PTL 6: WO 2020/206448A1
PTL 7: US 2013/0190399A1

### NON PATENT LITERATURE

NPTL 1: Dyerberg J et al. Am J Clin Nutr, 28(9): 958-66, 1975
NPTL 2: Dyerberg J et al. Lancet, 2(8081): 117-9, 1978
NPTL 3: Bang HO et al. Am J Clin Nutr, 33(12): 2657-61, 1980.
NPTL 4: Sherratt SCR et al. Biochemical and Biophysical Research Communications 496 (2018) 335-338
NPTL 5: Kiyomi Kikugawa, J. Lipid Nutr. Vol. 15, No. 1 (2006), 77-83
NPTL 6: Molecular Aspects of Medicine 64 (2018) 18-33

### SUMMARY OF INVENTION

### TECHNICAL PROBLEM

Various studies on methods for alleviating oxidative stress caused by reactive oxygen species have been reported. However, even currently, a method for sufficiently dealing with oxidative stress is strongly desired. An object of an aspect of the present invention is to provide a method for alleviating oxidative stress.

### SOLUTION TO PROBLEM

In an intensive search for a substance that suppresses oxidative stress caused by ROS, the present inventors have found that extension of the carbon chain of PUFA enables impartment or enhancement of an oxidative stress suppressing action, and a very-long-chain polyunsaturated fatty acid (VLC-PUFA) with extended chain length of PUFA is useful as an active ingredient of an oxidative stress inhibitor, leading to completion of the present invention.

The specification of the present application encompasses the disclosure of the following inventions.

[A-1] A composition for use in alleviation of oxidative stress, or treatment or prevention of an oxidative stress-related disorder, the composition comprising a very-long-chain polyunsaturated fatty acid (VLC-PUFA), a pharmaceutically functional derivative thereof or a pharmaceutically acceptable salt thereof as an active ingredient.

[A-2] The composition according to [A-1], wherein the pharmaceutically functional derivative is selected from the group consisting of an ester, an ether and an amide.

[A-3] The composition according to [A-1] or [A-2], wherein the pharmaceutically acceptable salt is selected from the group consisting of an inorganic salt and an organic salt.

[A-4] The composition according to any one of [A-1] to [A-3], wherein the very-long-chain polyunsaturated fatty acid is a fatty acid having 24 or more, 26 or more, 28 or more or 30 or more and 42 or less, 40 or less or 38 or less carbon atoms, containing 3 or more or 4 or more and 6 or less double bonds, and containing 0 or more and 1 or less, 2 or less or 3 or less hydroxy groups.

[A-5] The composition according to any one of [A-1] to [A-4], wherein the very-long-chain polyunsaturated fatty acid is a fatty acid represented by one of the following formulae: 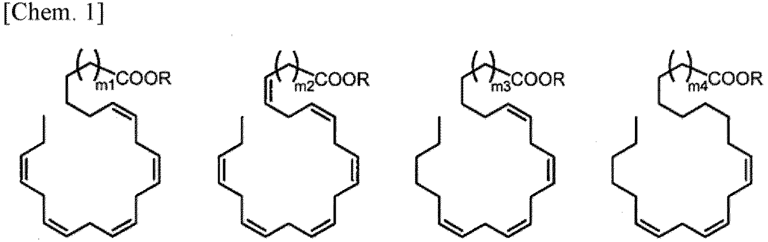 wherein m1, m2, m3 and m4 are each independently an integer selected from 4 to 22, 4 to 20, 6 to 20, 8 to 20, 10 to 20, 4 to 18, 6 to 18, 8 to 18 or 10 to 18, and R is a hydrogen atom.

[A-6] The composition according to any one of [A-1] to [A-5], wherein the very-long-chain polyunsaturated fatty acid is a very-long-chain fatty acid selected from C32:4n-6, C34:4n-6, C32:5n-3, C34:5n-3, C34:6n-3, C32:3n-6 and C34:3n-6.

[A-7] The composition according to any one of [A-1] to [A-6], wherein the very-long-chain polyunsaturated fatty acid is a very-long-chain polysaturated fatty acid of C32:4n-6 or C34:5n-3.

[A-8] The composition according to any one of [A-1] to [A-7], wherein the very-long-chain polyunsaturated fatty acid (VLC-PUFA), pharmaceutically functional derivative thereof or pharmaceutically acceptable salt thereof is an ethyl ester of a very-long-chain polyunsaturated fatty acid.

[A-9] The composition according to any one of [A-1] to [A-7], wherein the very-long-chain polyunsaturated fatty acid (VLC-PUFA), pharmaceutically functional derivative thereof or pharmaceutically acceptable salt thereof is a pharmaceutically acceptable basic amino acid salt of a very-long-chain polyunsaturated fatty acid.

[A-10] The composition according to [A-9], wherein the pharmaceutically acceptable basic amino acid salt of a very-long-chain polyunsaturated fatty acid is a lysine salt thereof.

[A-11] The composition according to any one of [A-1] to [A-10], for use in treatment or prevention of an oxidative stress-related disorder.

[A-12] The composition according to [A-11], wherein the oxidative stress-related disorder is selected from the group consisting of an eye disease, a neurological disease, an inflammatory reaction to oxidative stress, Down syndrome (DS), cancer, a cardiovascular disease, a respiratory disease, a lifestyle-related disease, a skin disorder, a gastrointestinal disease, a liver disease, a kidney disease, an autoimmune disease, an otorhinolaryngologic disease, sepsis, stress after organ transplantation, chronic fatigue syndrome and an age-related disease.

[A-13] The composition according to [A-11], wherein the oxidative stress-related disorder is selected from the group consisting of age-related macular degeneration (AMD), cataract, diabetic retinopathy, Alzheimer's disease (AD), mild cognitive impairment (MCI), amyotrophic lateral sclerosis (ALS), Parkinson's disease (PD), Down syndrome (DS), schizophrenia, bipolar disorder (BD) and synucleinopathy.

[A-14] The composition according to [A-11], wherein the oxidative stress-related disorder is selected from the group consisting of an inflammatory reaction to oxidative stress, retinal degeneration, atherosclerosis, amyotrophic lateral sclerosis (ALS), multiple sclerosis (MS), Friedreich ataxia, delayed dyskinesia, brain damage (for example, ischemia), photoaging of skin, reperfusion injury, brain stroke, myocardial infarction, hypertension, heart failure, epilepsy, hyperhomocysteinemia, physiological aging, sepsis and stress after organ transplantation.

[A-15] The composition according to any one of [A-1] to [A-14], wherein the oxidative stress-related disorder is a disorder caused by free radicals or reactive oxygen in a body.

[A-16] The composition according to any one of [A-1] to [A-15], wherein the oxidative stress-related disorder is a disorder related to the presence of ^{•}O₂⁻ (superoxide anion), H₂O₂ (hydrogen peroxide), *OH (hydroxyl radical), ROOH (organic hydroperoxide), RO* (alkoxyl radical), ROO' (peroxyl radical), HOCl (hypochlorous acid), OONO⁻ (peroxynitrite), NO', ¹O₂ (singlet oxygen), O₃ (ozone) or ^{•}NO₂ (nitrogen dioxide).

[A-17] The composition according to any one of [A-1] to [A-10], for use in alleviation of oxidative stress.

[A-18] The composition according to any one of [A-1] to [A-17], wherein the oxidative stress is a disorder caused by free radicals or reactive oxygen in a body.

[A-19] The composition according to any one of [A-1] to [A-18], wherein the oxidative stress is a disorder related to the presence of ^{•}O₂⁻ (superoxide anion), H₂O₂ (hydrogen peroxide), *OH (hydroxyl radical), ROOH (organic hydroperoxide), RO* (alkoxyl radical), ROO' (peroxyl radical), HOCl (hypochlorous acid), OONO⁻ (peroxynitrite), NO', ¹O₂ (singlet oxygen), O₃ (ozone) or ^{•}NO₂ (nitrogen dioxide).

[B-1] A method for treating or preventing an oxidative stress-related disorder, the method comprising administering an effective amount of a very-long-chain polyunsaturated fatty acid (VLC-PUFA), a pharmaceutically functional derivative thereof or a pharmaceutically acceptable salt thereof to a subject in need of the treatment or prevention.

[B-2] The method according to [B-1], wherein the pharmaceutically functional derivative is selected from the group consisting of an ester, an ether and an amide.

[B-3] The method according to [B-1] or [B-2], wherein the pharmaceutically acceptable salt is selected from the group consisting of an inorganic salt and an organic salt.

[B-4] The method according to any one of [B-1] to [B-3], wherein the very-long-chain polyunsaturated fatty acid is a fatty acid having 24 or more, 26 or more, 28 or more or 30 or more and 42 or less, 40 or less or 38 or less carbon atoms, containing 3 or more or 4 or more and 6 or less double bonds, and containing 0 or more and 1 or less, 2 or less or 3 or less hydroxy groups.

[B-5] The method according to any one of [B-1] to [B-4], wherein the very-long-chain polyunsaturated fatty acid is a fatty acid represented by one of the following formulae: 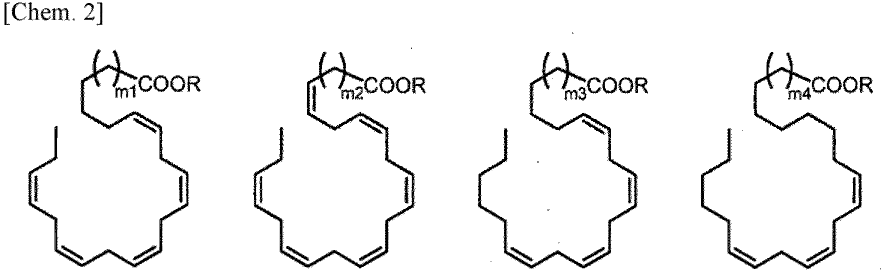 wherein m1, m2, m3 and m4 are each independently an integer selected from 4 to 22, 4 to 20, 6 to 20, 8 to 20, 10 to 20, 4 to 18, 6 to 18, 8 to 18 or 10 to 18, and R is a hydrogen atom.

[B-6] The method according to any one of [B-1] to [B-5], wherein the very-long-chain polyunsaturated fatty acid is a very-long-chain polyunsaturated fatty acid selected from C32:4n-6, C34:4n-6, C32:5n-3, C34:5n-3, C32:6n-3, C34:6n-3, C32:3n-6 and C34:3n-6.

[B-7] The method according to any one of [B-1] to [B-6], wherein the very-long-chain polyunsaturated fatty acid is a very-long-chain polysaturated fatty acid of C32:4n-6 or C34:5n-3.

[B-8] The method according to any one of [B-1] to [B-7], wherein the very-long-chain polyunsaturated fatty acid (VLC-PUFA), pharmaceutically functional derivative thereof or pharmaceutically acceptable salt thereof is an ethyl ester of a very-long-chain polyunsaturated fatty acid.

[B-9] The method according to any one of [B-1] to [B-7], wherein the very-long-chain polyunsaturated fatty acid (VLC-PUFA), pharmaceutically functional derivative thereof or pharmaceutically acceptable salt thereof is a pharmaceutically acceptable basic amino acid salt of a very-long-chain polyunsaturated fatty acid.

[B-10] The method according to [B-9], wherein the pharmaceutically acceptable basic amino acid salt of a very-long-chain polyunsaturated fatty acid is a lysine salt thereof.

[B-11] The method according to any one of [B-1] to [B-10], wherein the oxidative stress-related disorder is selected from the group consisting of an eye disease, a neurological disease, an inflammatory reaction to oxidative stress, Down syndrome (DS), cancer, a cardiovascular disease, a respiratory disease, a lifestyle-related disease, a skin disorder, a gastrointestinal disease, a liver disease, a kidney disease, an autoimmune disease, an otorhinolaryngologic disease, sepsis, stress after organ transplantation, chronic fatigue syndrome and an age-related disease.

[B-12] The method according to any one of [B-1] to [B-11], wherein the oxidative stress-related disorder is selected from the group consisting of age-related macular degeneration (AMD), cataract, diabetic retinopathy, Alzheimer's disease (AD), mild cognitive impairment (MCI), amyotrophic lateral sclerosis (ALS), Parkinson's disease (PD), Down syndrome (DS), schizophrenia, bipolar disorder (BD) and synucleinopathy.

[B-13] The method according to any one of [B-1] to [B-11], wherein the oxidative stress-related disorder is selected from the group consisting of an inflammatory reaction to oxidative stress, retinal degeneration, atherosclerosis, amyotrophic lateral sclerosis (ALS), multiple sclerosis (MS), Friedreich ataxia, delayed dyskinesia, brain damage (for example, ischemia), photoaging of skin, reperfusion injury, brain stroke, myocardial infarction, hypertension, heart failure, epilepsy, hyperhomocysteinemia, physiological aging, sepsis and stress after organ transplantation.

[B-14] The method according to any one of [B-1] to [B-13], wherein the oxidative stress-related disorder is a disorder caused by free radicals or reactive oxygen in a body.

[B-15] The method according to any one of [B-1] to [B-14], wherein the oxidative stress-related disorder is a disorder related to the presence of ^{•}O₂⁻ (superoxide anion), H₂O₂ (hydrogen peroxide), *OH (hydroxyl radical), ROOH (organic hydroperoxide), RO* (alkoxyl radical), ROO' (peroxyl radical), HOCl (hypochlorous acid), OONO⁻ (peroxynitrite), NO', ¹O₂ (singlet oxygen), O₃ (ozone) or ^{•}NO₂ (nitrogen dioxide).

[C-1] A method for alleviating oxidative stress, the method comprising administering an effective amount of a very-long-chain polyunsaturated fatty acid (VLC-PUFA), a pharmaceutically functional derivative thereof or a pharmaceutically acceptable salt thereof to a subject in need of the treatment or prevention.

[C-2] The method according to [C-1], wherein the pharmaceutically functional derivative is selected from the group consisting of an ester, an ether and an amide.

[C-3] The method according to [C-1] or [C-2], wherein the pharmaceutically acceptable salt is selected from the group consisting of an inorganic salt and an organic salt.

[C-4] The method according to any one of [C-1] to [C-3], wherein the very-long-chain polyunsaturated fatty acid is a fatty acid having 24 or more, 26 or more, 28 or more or 30 or more and 42 or less, 40 or less or 38 or less carbon atoms, containing 3 or more or 4 or more and 6 or less double bonds, and containing 0 or more and 1 or less, 2 or less or 3 or less hydroxy groups.

[C-5] The method according to any one of [C-1] to [C-4], wherein the very-long-chain polyunsaturated fatty acid is a fatty acid represented by one of the following formulae: 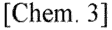 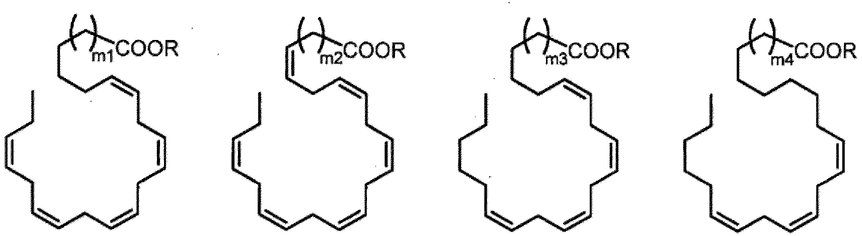 wherein m1, m2, m3 and m4 are each independently an integer selected from 4 to 22, 4 to 20, 6 to 20, 8 to 20, 10 to 20, 4 to 18, 6 to 18, 8 to 18 or 10 to 18, and R is a hydrogen atom.

[C-6] The method according to any one of [C-1] to [C-5], wherein the very-long-chain polyunsaturated fatty acid is a very-long-chain polyunsaturated fatty acid selected from C32:4n-6, C34:4n-6, C32:5n-3, C34:5n-3, C32:6n-3, C34:6n-3, C32:3n-6 and C34:3n-6.

[C-7] The method according to any one of [C-1] to [C-6], wherein the very-long-chain polyunsaturated fatty acid is a very-long-chain polysaturated fatty acid of C32:4n-6 or C34:5n-3.

[C-8] The method according to any one of [C-1] to [C-7], wherein the very-long-chain polyunsaturated fatty acid (VLC-PUFA), pharmaceutically functional derivative thereof or pharmaceutically acceptable salt thereof is an ethyl ester of a very-long-chain polyunsaturated fatty acid.

[C-9] The method according to any one of [C-1] to [C-7], wherein the very-long-chain polyunsaturated fatty acid (VLC-PUFA), pharmaceutically functional derivative thereof or pharmaceutically acceptable salt thereof is a pharmaceutically acceptable basic amino acid salt of a very-long-chain polyunsaturated fatty acid.

[C-10] The method according to [C-9], wherein the pharmaceutically acceptable basic amino acid salt of a very-long-chain polyunsaturated fatty acid is a lysine salt thereof.

[C-11] The method according to any one of [C-1] to [C-10], wherein the oxidative stress-related disorder is selected from the group consisting of an eye disease, a neurological disease, an inflammatory reaction to oxidative stress, Down syndrome (DS), cancer, a cardiovascular disease, a respiratory disease, a lifestyle-related disease, a skin disorder, a gastrointestinal disease, a liver disease, a kidney disease, an autoimmune disease, an otorhinolaryngologic disease, sepsis, stress after organ transplantation, chronic fatigue syndrome and an age-related disease.

[C-12] The method according to any one of [C-1] to [C-11], wherein the oxidative stress-related disorder is selected from the group consisting of age-related macular degeneration (AMD), cataract, diabetic retinopathy, Alzheimer's disease (AD), mild cognitive impairment (MCI), amyotrophic lateral sclerosis (ALS), Parkinson's disease (PD), Down syndrome (DS), schizophrenia, bipolar disorder (BD) and synucleinopathy.

[C-13] The method according to any one of [C-1] to [C-12], wherein the oxidative stress-related disorder is selected from the group consisting of an inflammatory reaction to oxidative stress, retinal degeneration, atherosclerosis, amyotrophic lateral sclerosis (ALS), multiple sclerosis (MS), Friedreich ataxia, delayed dyskinesia, brain damage (for example, ischemia), photoaging of skin, reperfusion injury, brain stroke, myocardial infarction, hypertension, heart failure, epilepsy, hyperhomocysteinemia, physiological aging, sepsis and stress after organ transplantation.

[C-14] The method according to any one of [C-1] to [C-13], wherein the oxidative stress is a disorder caused by free radicals or reactive oxygen in a body.

[C-15] The method according to any one of [C-1] to [C-14], wherein the oxidative stress is a disorder related to the presence of ^{•}O₂⁻ (superoxide anion), H₂O₂ (hydrogen peroxide), *OH (hydroxyl radical), ROOH (organic hydroperoxide), RO* (alkoxyl radical), ROO' (peroxyl radical), HOCl (hypochlorous acid), OONO⁻ (peroxynitrite), NO', ¹O₂ (singlet oxygen), O₃ (ozone) or ^{•}NO₂ (nitrogen dioxide).

[D-1] A composition for use in alleviation of oxidative stress, or treatment or prevention of an oxidative stress-related disorder, the composition comprising a very-long-chain polyunsaturated fatty acid (VLC-PUFA), or a salt thereof or an ester thereof as an active ingredient, the ester being a C₁₋₆ alkyl ester or glyceride.

[D-2] The composition according to [D-1], wherein the very-long-chain polyunsaturated fatty acid is a fatty acid having 24 or more, 26 or more, 28 or more or 30 or more and 40 or less or 38 or less carbon atoms, and containing 3 or more or 4 or more and 6 or less double bonds.

[D-3] The composition according to [D-1] or [D-2], wherein the very-long-chain polyunsaturated fatty acid has 3 to 6 double bonds, and has a chemical structure of 6n-3, 5n-3, 4n-6 or 3n-6 which is the same as that of docosahexaenoic acid (DHA), eicosapentaenoic acid (EPA), arachidonic acid (ARA) or dihomo-γ-linoleic acid (DGLA).

[D-4] The composition according to any one of [D-1] to [D-3], wherein the very-long-chain polyunsaturated fatty acid is selected from the very-long-chain polyunsaturated fatty acids of C32:4n-6, C34:4n-6, C32:5n-3, C34:5n-3, C34:6n-3, C32:3n-6 and C34:3n-6.

[D-5] The composition according to any one of [D-1] to [D-4], wherein the very-long-chain polyunsaturated fatty acid is a very-long-chain polyunsaturated fatty acid of C32:4n-6 or C34:5n-3.

[D-6] The composition according to any one of [D-1] to [D-5], comprising a very-long-chain polyunsaturated fatty acid ethyl ester.

[D-7] The composition according to any one of [D-1] to [D-6], for use in treatment or prevention of an oxidative stress-related disorder.

[D-8] The composition according to [D-7], wherein the oxidative stress-related disorder is selected from the group consisting of an eye disease, a neurological disease, an inflammatory reaction to oxidative stress, Down syndrome (DS), cancer, a cardiovascular disease, a respiratory disease, a lifestyle-related disease, a skin disorder, a gastrointestinal disease, a liver disease, a kidney disease, an autoimmune disease, an otorhinolaryngologic disease, sepsis, stress after organ transplantation, chronic fatigue syndrome and an age-related disease.

[D-9] The composition according to [D-7], wherein the oxidative stress-related disorder is selected from the group consisting of age-related macular degeneration (AMD), cataract, diabetic retinopathy, Alzheimer's disease (AD), mild cognitive impairment (MCI), amyotrophic lateral sclerosis (ALS), Parkinson's disease (PD), Down syndrome (DS), schizophrenia, bipolar disorder (BD) and synucleinopathy.

[D-10] The composition according to [D-7], wherein the oxidative stress-related disorder is selected from the group consisting of an inflammatory reaction to oxidative stress, retinal degeneration, atherosclerosis, amyotrophic lateral sclerosis (ALS), multiple sclerosis (MS), Friedreich ataxia, delayed dyskinesia, brain damage (for example, ischemia), photoaging of skin, reperfusion injury, brain stroke, myocardial infarction, hypertension, heart failure, epilepsy, hyperhomocysteinemia, physiological aging, sepsis and stress after organ transplantation.

[D-11] The composition according to any one of [D-1] to [D-10], wherein the oxidative stress-related disorder is a disorder caused by free radicals or reactive oxygen in a body.

[D-12] The composition according to any one of [D-1] to [D-11], wherein the oxidative stress-related disorder is a disorder related to the presence of ^{•}O₂⁻ (superoxide anion), H₂O₂ (hydrogen peroxide), *OH (hydroxyl radical), ROOH (organic hydroperoxide), RO* (alkoxyl radical), ROO' (peroxyl radical), HOCl (hypochlorous acid), OONO⁻ (peroxynitrite) or NO'.

[D-13] The composition according to any one of [D-1] to [D-6], for use in alleviation of oxidative stress.

[D-14] The composition according to any one of [D-1] to [D-13], wherein the oxidative stress is a disorder caused by free radicals or reactive oxygen in a body.

[D-15] The composition according to any one of [D-1] to [D-14], wherein the oxidative stress is a disorder related to the presence of ^{•}O₂⁻ (superoxide anion), H₂O₂ (hydrogen peroxide), *OH (hydroxyl radical), ROOH (organic hydroperoxide), RO* (alkoxyl radical), ROO' (peroxyl radical), HOCl (hypochlorous acid), OONO⁻ (peroxynitrite) or NO'.

[D-16] The composition according to any one of [D-1] to [D-5] and [D-7]-[D-15], wherein the very-long-chain polyunsaturated fatty acid (VLC-PUFA), salt thereof or ester thereof is a pharmaceutically acceptable basic amino acid salt of a very-long-chain polyunsaturated fatty acid.

[D-17] The composition according to [D-16], wherein the pharmaceutically acceptable basic amino acid salt of a very-long-chain polyunsaturated fatty acid is a lysine salt thereof.

[E-1] A method for treating or preventing an oxidative stress-related disorder, the method comprising administering an effective amount of a very-long-chain polyunsaturated fatty acid (VLC-PUFA), a salt thereof or an ester thereof to a subject in need of the treatment or prevention, the ester being a C₁₋₆ alkyl ester or glyceride.

[E-2] The method according to [E-1], wherein the very-long-chain polyunsaturated fatty acid is a fatty acid having 24 or more, 26 or more, 28 or more or 30 or more and 40 or less or 38 or less carbon atoms, and containing 3 or more or 4 or more and 6 or less double bonds.

[E-3] The method according to [E-1] or [E-2], wherein the very-long-chain polyunsaturated fatty acid has 3 to 6 double bonds, and has a chemical structure of 6n-3, 5n-3, 4n-6 or 3n-6 which is the same as that of docosahexaenoic acid (DHA), eicosapentaenoic acid (EPA), arachidonic acid (ARA) or dihomo-γ-linoleic acid (DGLA).

[E-4] The method according to any one of [E-1] to [E-3], wherein the very-long-chain polyunsaturated fatty acid is selected from the very-long-chain polyunsaturated fatty acids of C32:4n-6, C34:4n-6, C32:5n-3, C34:5n-3, C32:6n-3, C34:6n-3, C32:3n-6 and C34:3n-6.

[E-5] The method according to any one of [E-1] to [E-4], wherein the very-long-chain polyunsaturated fatty acid is a very-long-chain polyunsaturated fatty acid of C32:4n-6 or C34:5n-3.

[E-6] The method according to any one of [E-1] to [E-5], comprising a very-long-chain polyunsaturated fatty acid ethyl ester.

[E-7] The method according to any one of [E-1] to [E-6], wherein the oxidative stress-related disorder is selected from the group consisting of an eye disease, a neurological disease, an inflammatory reaction to oxidative stress, Down syndrome (DS), cancer, a cardiovascular disease, a respiratory disease, a lifestyle-related disease, a skin disorder, a gastrointestinal disease, a liver disease, a kidney disease, an autoimmune disease, an otorhinolaryngologic disease, sepsis, stress after organ transplantation, chronic fatigue syndrome and an age-related disease.

[E-8] The method according to any one of [E-1] to [E-6], wherein the oxidative stress-related disorder is selected from the group consisting of age-related macular degeneration (AMD), cataract, diabetic retinopathy, Alzheimer's disease (AD), mild cognitive impairment (MCI), amyotrophic lateral sclerosis (ALS), Parkinson's disease (PD), Down syndrome (DS), schizophrenia, bipolar disorder (BD) and synucleinopathy.

[E-9] The method according to any one of [E-1] to [E-6], wherein the oxidative stress-related disorder is selected from the group consisting of an inflammatory reaction to oxidative stress, retinal degeneration, atherosclerosis, amyotrophic lateral sclerosis (ALS), multiple sclerosis (MS), Friedreich ataxia, delayed dyskinesia, brain damage (for example, ischemia), photoaging of skin, reperfusion injury, brain stroke, myocardial infarction, hypertension, heart failure, epilepsy, hyperhomocysteinemia, physiological aging, sepsis and stress after organ transplantation.

[E-10] The method according to any one of [E-1] to [E-9], wherein the oxidative stress-related disorder is a disorder caused by free radicals or reactive oxygen in a body.

[E-11] The method according to any one of [E-1] to [E-10], wherein the oxidative stress-related disorder is a disorder related to the presence of ^{•}O₂⁻ (superoxide anion), H₂O₂ (hydrogen peroxide), *OH (hydroxyl radical), ROOH (organic hydroperoxide), RO* (alkoxyl radical), ROO' (peroxyl radical), HOCl (hypochlorous acid), OONO⁻ (peroxynitrite) or NO'.

[E-12] The method according to any one of [E-1] to [E-5] and [E-7] to [E-11], wherein the very-long-chain polyunsaturated fatty acid (VLC-PUFA), salt thereof or ester thereof is a pharmaceutically acceptable basic amino acid salt of a very-long-chain polyunsaturated fatty acid.

[E-13] The method according to [E-12], wherein the pharmaceutically acceptable basic amino acid salt of a very-long-chain polyunsaturated fatty acid is a lysine salt thereof.

[F-1] A method for alleviating oxidative stress, the method comprising administering an effective amount of a very-long-chain polyunsaturated fatty acid (VLC-PUFA), a salt thereof or an ester thereof to a subject in need of the treatment or prevention, the ester being a C₁₋₆ alkyl ester or glyceride.

[F-2] The method according to [F-1], wherein the very-long-chain polyunsaturated fatty acid is a fatty acid having 24 or more, 26 or more, 28 or more or 30 or more and 40 or less or 38 or less carbon atoms, and containing 3 or more or 4 or more and 6 or less double bonds.

[F-3] The method according to [F-1] or [F-2], wherein the very-long-chain polyunsaturated fatty acid has 3 to 6 double bonds, and has a chemical structure of 6n-3, 5n-3, 4n-6 or 3n-6 which is the same as that of docosahexaenoic acid (DHA), eicosapentaenoic acid (EPA), arachidonic acid (ARA) or dihomo-γ-linoleic acid (DGLA).

[F-4] The method according to any one of [F-1] to [F-3], wherein the very-long-chain polyunsaturated fatty acid is selected from the very-long-chain polyunsaturated fatty acids of C32:4n-6, C34:4n-6, C32:5n-3, C34:5n-3, C32:6n-3, C34 6n-3, C32:3n-6 and C34:3n-6.

[F-5] The method according to any one of [F-1] to [F-4], wherein the very-long-chain polyunsaturated fatty acid is a very-long-chain polyunsaturated fatty acid of C32:4n-6 or C34:5n-3.

[F-6] The method according to any one of [F-1] to [F-5], comprising a very-long-chain polyunsaturated fatty acid ethyl ester.

[F-7] The method according to any one of [F-1] to [F-6], wherein the oxidative stress-related disorder is selected from the group consisting of an eye disease, a neurological disease, an inflammatory reaction to oxidative stress, Down syndrome (DS), cancer, a cardiovascular disease, a respiratory disease, a lifestyle-related disease, a skin disorder, a gastrointestinal disease, a liver disease, a kidney disease, an autoimmune disease, an otorhinolaryngologic disease, sepsis, stress after organ transplantation, chronic fatigue syndrome and an age-related disease.

[F-8] The method according to any one of [F-1] to [F-6], wherein the oxidative stress-related disorder is selected from the group consisting of age-related macular degeneration (AMD), cataract, diabetic retinopathy, Alzheimer's disease (AD), mild cognitive impairment (MCI), amyotrophic lateral sclerosis (ALS), Parkinson's disease (PD), Down syndrome (DS), schizophrenia, bipolar disorder (BD) and synucleinopathy.

[F-9] The method according to any one of [F-1] to [F-6], wherein the oxidative stress-related disorder is selected from the group consisting of an inflammatory reaction to oxidative stress, retinal degeneration, atherosclerosis , amyotrophic lateral sclerosis (ALS), multiple sclerosis (MS), Friedreich ataxia, delayed dyskinesia, brain damage (for example, ischemia), photoaging of skin, reperfusion injury, brain stroke, myocardial infarction, hypertension, heart failure, epilepsy, hyperhomocysteinemia, physiological aging, sepsis and stress after organ transplantation.

[F-10] The method according to any one of [F-1] to [F-9], wherein the oxidative stress is a disorder caused by free radicals or reactive oxygen in a body.

[F-11] The method according to any one of [F-1] to [F-10], wherein the oxidative stress is a disorder related to the presence of ^{•}O₂⁻ (superoxide anion), H₂O₂ (hydrogen peroxide), *OH (hydroxyl radical), ROOH (organic hydroperoxide), RO* (alkoxyl radical), ROO' (peroxyl radical), HOCl (hypochlorous acid), OONO⁻ (peroxynitrite) or NO'.

[F-12] The composition according to any one of [F-1] to [F-5] and [F-7] to [F-11], wherein the very-long-chain polyunsaturated fatty acid (VLC-PUFA), salt thereof or ester thereof is a pharmaceutically acceptable basic amino acid salt of a very-long-chain polyunsaturated fatty acid.

[F-13] The composition according to [F-12], wherein the pharmaceutically acceptable basic amino acid salt of a very-long-chain polyunsaturated fatty acid is a lysine salt thereof.

An aspect of the present invention provides a very-long-chain polyunsaturated fatty acid (VLC-PUFA), a pharmaceutically functional derivative thereof or a pharmaceutically acceptable salt thereof for use in treatment or prevention of an oxidative stress-related disorder.

An aspect of the present invention provides use of a very-long-chain polyunsaturated fatty acid (VLC-PUFA), a pharmaceutically functional derivative thereof or a pharmaceutically acceptable salt thereof for producing a composition for treatment or prevention of an oxidative stress-related disorder.

### ADVANTAGEOUS EFFECTS OF INVENTION

According to the present invention, a method for alleviating oxidative stress, and a method for treating or preventing a disease or a symptom caused by oxidative stress are provided.

### BRIEF DESCRIPTION OF DRAWINGS

Fig. 1 is a graph showing the results of evaluating oxidative stress by quantifying the amount of a reactive oxygen species.
Fig. 2 is a graph showing the results of evaluating an effect of protection from oxidative stress in Example 2.
Fig. 3 is a graph showing the results of evaluating an effect of protection from oxidative stress in Example 3.
Fig. 4 is a graph showing the results of evaluating an effect of protection from oxidative stress in Example 4.

### DESCRIPTION OF EMBODIMENTS

### Definitions

Described below are the definitions of various terms used to describe the present invention. These definitions are applied to terms used throughout the present specification and claims unless otherwise specified separately or as a part of a larger group in a specific case.

Unless otherwise defined, technical terms and scientific terms used in the present specification generally have the same meanings as those that are generally understood by a person skilled in the art to which the present invention belongs. In general, nomenclatures and experimental procedures in cell culture, molecular genetics, organic chemistry and peptide chemistry, which are used in the present specification, are well known and generally used in the art.

As used in the present specification, the term "pharmaceutically acceptable" refers to a relatively non-toxic material such as that for a carrier or diluent, which does not impair the biological activity or characteristics of an active ingredient (that is, such a material can be administered to a subject without causing an undesirable biological action or undergoing a harmful reaction with any of components of a composition containing the material).

As used in the present specification, the term "pharmaceutically acceptable salt" refers to a derivative of the disclosed very-long-chain polyunsaturated fatty acid, which is obtained by converting an acid or base moiety present in the very-long-chain polyunsaturated fatty acid or a derivative thereof into a salt form of the moiety. Examples of the pharmaceutically acceptable salt include, but are not limited to, inorganic salts or organic salts of acidic residues such as carboxylic acids; mineral acid salts or organic acid salts of basic residues such as amines; and salts similar thereto. As the inorganic salt of an acidic residue such as a carboxylic acid, alkali metal salts such as potassium salts and sodium salts, and salts of Group II elements such as calcium salts and magnesium salts are exemplified. As the organic salt of an acidic residue such as a carboxylic acid, amine salts such as lysine salts, arginine salts, omithine salts, choline salts, meglumine salts, benzathine salts and tromethamine salts are exemplified.

Examples of the pharmaceutically acceptable salt in the present invention include non-toxic salts of a very-long-chain polyunsaturated fatty acid formed from a well-known non-toxic inorganic salt or organic salt. The pharmaceutically acceptable salt in the present invention can be synthesized from a very-long-chain polyunsaturated fatty acid having a basic or acidic moiety by a well-known chemical method. In general, such a salt can be prepared by reacting a free acid or a base of the very-long-chain polyunsaturated fatty acid with a stoichiometric amount of a proper base or acid in water, an organic solvent or a mixture thereof. Generally, nonaqueous medium such as ether, ethyl acetate, ethanol, isopropanol or acetonitrile is preferable. The lists of appropriate salts are found in Remington's Pharmaceutical Sciences, 17th ed., Mack Publishing Company, Easton, Pa., 1985, p. 1418 and Journal of Pharmaceutical Science, 66, 2 (1977), each of which is incorporated herein by reference in its entirety.

The "pharmaceutically functional derivative" of a very-long-chain polyunsaturated fatty acid as defined in the present specification includes derivatives having or providing the same biological functions and/or activity as those of any of the very-long-chain polyunsaturated fatty acids of the present invention. Therefore, this term includes esters, ethers, amides and prodrugs of the very-long-chain polyunsaturated fatty acid.

The "ether" of the very-long-chain polyunsaturated fatty acid includes derivatives obtained by converting a hydroxy group present in a very-long-chain polyunsaturated fatty acid into ether, and derivatives obtained by reducing a carboxy group (-CO₂H) of a very-long-chain polyunsaturated fatty acid to a hydroxymethyl group (-CH₂OH) and converting the hydroxymethyl group into ether.

The "prodrug" of a very-long-chain polyunsaturated fatty acid includes compounds which are, after oral or parenteral administration, metabolized *in vivo* to form an experimentally detectable amount of the very-long-chain polyunsaturated fatty acid within a predetermined time (for example, within a dosing interval of 6 to 24 hours (that is, one to four times a day)).

The prodrug of a very-long-chain polyunsaturated fatty acid can be prepared by modifying a functional group present in the very-long-chain polyunsaturated fatty acid so as to cleave the modification *in vivo* when the prodrug is administered to a subject. The modification is typically achieved by protecting a functional group of the very-long-chain polyunsaturated fatty acid with a prodrug substituent. For example, a hydroxy, amino, sulfhydryl, carboxy or carbonyl group of a very-long-chain polyunsaturated fatty acid can be protected with any group that can be cleaved *in vivo* for regenerating the free hydroxy, amino, sulfhydryl, carboxy or carbonyl group.

Examples of the prodrug include, but are not limited to, esters and carbamates of a hydroxy functional group, and ester groups, N-acyl derivatives and N-Mannich bases of a carboxy functional group. General information about prodrugs is described in, for example, Bundegaard, H., Design of Prodrugs, p. I-92, Elsevier, New York-Oxford (1985).

In the present specification, the very-long-chain polyunsaturated fatty acid, a pharmaceutically functional derivative thereof and a pharmaceutically acceptable salt thereof may be collectively, or in part, referred to as an "active ingredient".

For writing a fatty acid, a numerical notation may be used in which the number of carbon atoms, the number of double bonds and the location of the double bond are indicated in a simple form respectively using numbers and alphabets. For example, a saturated fatty acid having 20 carbon atoms is written as "C20:0", a monounsaturated fatty acid having 18 carbon atoms is written as "C18:1" or the like, and eicosapentaenoic acid is written as "C20:5n-3" or the like. Here, "n-3" is also written in the form of ω-3, which indicates that the linkage position of the first double bond from the rearmost carbon (ω) toward carboxy is the 3rd. In this notation, all double bonds are in the cis-form, and the double bonds of a polyunsaturated fatty acid have a methylene-interrupted structure in which one methylene is sandwiched between the double bond and the next double bond. This notation is well known to a person skilled in the art, and fatty acids written in accordance with this notation can be easily identified by a person skilled in the art.

In the present specification, the very-long-chain polyunsaturated fatty acid is not particularly limited as long as it is a fatty acid having 24 or more carbon atoms and containing 3 or more double bonds. In an aspect of the present invention, the number of carbon atoms in the very-long-chain polyunsaturated fatty acid is 24 to 42, 24 to 40, 26 to 40, 28 to 40, 30 to 40, 24 to 38, 26 to 38, 28 to 38 or 30 to 38. In an embodiment of the present invention, the very-long-chain polyunsaturated fatty acid has 4 to 6 double bonds. In an embodiment of the present invention, the very-long-chain polyunsaturated fatty acid has 3 to 6 double bonds. In an aspect of the present invention, the very-long-chain polyunsaturated fatty acid has 0 to 3 hydroxy groups. In an aspect of the present invention, the very-long-chain polyunsaturated fatty acid has 0 to 2 hydroxy groups.

In an embodiment of the present invention, the very-long-chain polyunsaturated fatty acid has 3 to 6 cis-form double bonds that are sequentially positioned with one methylene sandwiched between the double bonds. In an embodiment of the present invention, the very-long-chain polyunsaturated fatty acid has 3 to 6 double bonds that are sequentially positioned with one methylene sandwiched in a chemical structure which is the same as that of docosahexaenoic acid, eicosapentaenoic acid, arachidonic acid or dihomo-γ-linoleic acid.

In an aspect of the present invention, the very-long-chain polyunsaturated fatty acid is a n-3 or n-6 very-long-chain polyunsaturated fatty acid (n-3 VLC-PUFA or n-6 VLC-PUFA) containing 24 to 42 carbon atoms (C24-C42). Examples thereof include C32:6n-3 (32 carbon atoms, 6 double bonds, ω-3), C34:6n-3, C32:5n-3 and C34:5n-3.

In an aspect of the present invention, the very-long-chain polyunsaturated fatty acid is a fatty acid represented by the following formula: 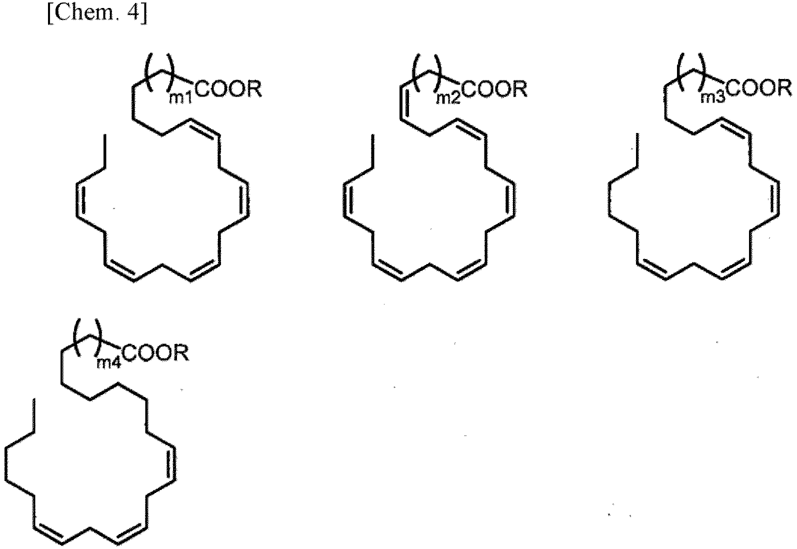 wherein m1, m2, m3 and m4 are independently an integer selected from 4 to 22, 4 to 20, 6 to 20, 8 to 20, 10 to 20, 4 to 18, 6 to 18, 8 to 18 or 10 to 18, and R is a hydrogen atom.

In an aspect of the present invention, the very-long-chain polyunsaturated fatty acid, a salt thereof or an ester thereof is a fatty acid represented by the following formula, a salt thereof, or an ester thereof. 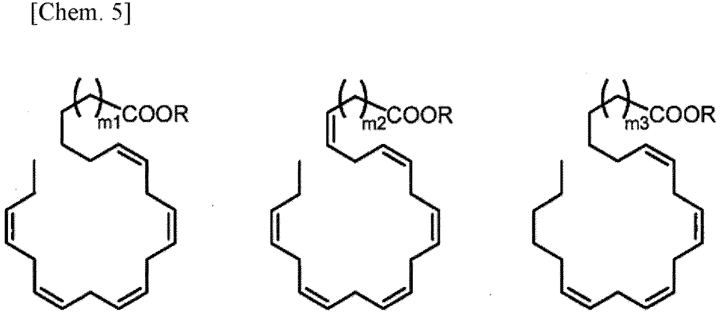 wherein m1, m2 and m3 are independently an integer selected from 4 to 22, 4 to 20, 6 to 20, 8 to 20, 10 to 20, 4 to 18, 6 to 18, 8 to 18 or 10 to 18, and R is a hydrogen atom, a cation such as an alkali metal ion, or an ester-forming group such as a C₁₋₆ alkyl.

In an aspect of the present invention, as the very-long-chain polyunsaturated fatty acid (VLC-PUFA), pharmaceutically functional derivative thereof or pharmaceutically acceptable salt thereof, a very-long-chain polyunsaturated fatty acid can be used.

In an embodiment of the present invention, as the very-long-chain polyunsaturated fatty acid (VLC-PUFA), pharmaceutically functional derivative thereof or pharmaceutically acceptable salt thereof, a pharmaceutically functional derivative of a very-long-chain polyunsaturated fatty acid can be used. The pharmaceutically functional derivative of a very-long-chain polyunsaturated fatty acid include esters, ethers, amides, and prodrugs, with esters being particularly preferable. The ester derivative is not particularly limited, and includes alkyl esters, and glycerides. The alkyl ester is, for example, a C₁₋₆ alkyl ester, and preferably an ethyl ester. The glyceride may be triglyceride, diglyceride or monoglyceride, and at least one of the constituent fatty acids of the glyceride is a very-long-chain polyunsaturated fatty acid. Preferably, the ester of the very-long-chain polyunsaturated fatty acid is an ethyl ester.

The ether derivative of the very-long-chain polyunsaturated fatty acid is not particularly limited, and includes alkyl ethers, and alkenyl ethers. The alkyl ether is, for example, a C₁₋₆ alkyl ester. The alkenyl ether is, for example, a C₂₋₆ alkenyl ether containing one double bond.

The amide derivative of the very-long-chain polyunsaturated fatty acid is not particularly limited, and includes amides (-CONH₂), N-alkylamides, and N,N-dialkylamides. The N-alkylamide is, for example, a N-C₁₋₆ alkylamide. The N,N-dialkylamide is, for example, a N,N-diC₁₋₆ alkylamide.

In an aspect of the present invention, as the very-long-chain polyunsaturated fatty acid (VLC-PUFA), pharmaceutically functional derivative thereof or pharmaceutically acceptable salt thereof, a pharmaceutically acceptable salt of a very-long-chain polyunsaturated fatty acid or a derivative thereof can be used. The pharmaceutically acceptable salt of a very-long-chain polyunsaturated fatty acid or a derivative thereof includes inorganic salts and organic salts. As the inorganic salt, alkali metal salts such as potassium salts and sodium salts, and salts of Group II elements such as calcium salts and magnesium salts are exemplified. As the organic salt, amine salts such as lysine salts, arginine salts, omithine salts, choline salts, meglumine salts, benzathine salts and tromethamine salts are exemplified.

In an aspect of the present invention, as the very-long-chain polyunsaturated fatty acid (VLC-PUFA), pharmaceutically functional derivative thereof or pharmaceutically acceptable salt thereof, a pharmaceutically acceptable salt of a very-long-chain polyunsaturated fatty acid can be used. The pharmaceutically acceptable salt of a very-long-chain polyunsaturated fatty acid is preferably a basic amino acid salt such as a lysine salt, an arginine salt or an ornithine salt, and more preferably a basic amino acid salt such as a lysine salt.

In an aspect of the present invention, a very-long-chain polyunsaturated fatty acid, a salt thereof or an ester thereof can be used. Here, the ester is not particularly limited, and examples thereof include C₁₋₆ alkyl esters, and glycerides. Examples of the glyceride may be triglyceride, diglyceride or monoglyceride, and at least one of constituent fatty acids of the glyceride is a very-long-chain polyunsaturated fatty acid. Preferably, the ester of a very-long-chain polyunsaturated fatty acid is an ethyl ester. As the salt of a very-long-chain polyunsaturated fatty acid, alkali metal salts such as potassium salts and sodium salts are exemplified.

In an embodiment of the present invention, an active ingredient such as a very-long-chain polyunsaturated fatty acid can be acquired by chemical synthesis using a long-chain polyunsaturated fatty acid (for example, docosahexaenoic acid, eicosapentaenoic acid or arachidonic acid) as a starting material. In an embodiment of the present invention, an active ingredient such as a very-long-chain polyunsaturated fatty acid can be acquired by collecting products obtained by biosynthesis in cells containing an elongase enzyme. For example, an active ingredient such as a very-long-chain polyunsaturated fatty acid can be acquired by concentration and purification from, for example, fish oil, cuttlefish oil, alga oil or krill oil.

An active ingredient such as a very-long-chain polyunsaturated fatty acid can be acquired by a known method described in, for example, Japanese Translation of PCT International Application Publication No. 2018-506584, US 2013/0190399A1, WO 2017/002353A1 or Japanese Translation of PCT International Application Publication No. 2018-506584.

In an aspect of the present invention, the composition according to the present invention can be used as a therapeutic agent or a prophylactic agent for oxidative stress-related disorders, in particular, diseases caused by oxidative stress. In the present specification, the oxidative stress refers to, for example, a state of stress in which endogenous or exogenous reactive oxygen species (superoxide anions, hydroxyl radicals, hydrogen peroxide, hypochlorous acid, nitrogen monoxide and the like) are excessively produced, so that a living body cannot sufficiently treat the reactive oxygen species. The reactive oxygen species related to oxidative stress-related disorders is not particularly limited, and examples thereof include ^{•}O2⁻ (superoxide anion), H₂O₂ (hydrogen peroxide), *OH (hydroxyl radical), ROOH (organic hydroperoxide), RO* (alkoxyl radical), ROO' (peroxyl radical), HOCl (hypochlorous acid), OONO⁻ (peroxynitrite), NO', ¹O₂ (singlet oxygen), O₃ (ozone) and ^{•}NO₂ (nitrogen dioxide).

In an aspect of the present invention, the disease caused by oxidative stress according to the present invention is not particularly limited, and examples thereof include
eye diseases (retinal degeneration (age-related macular degeneration (AMD) (for example, age-related maculopathy based on oxidative stress), diabetic retinopathy and the like), cataract, dry eye, and the like),
neurological diseases (Alzheimer's disease, Parkinson's disease, synucleinopathy, mild cognitive impairment, amyotrophic lateral sclerosis (ALS), multiple sclerosis (MS), Friedreich ataxia, epilepsy, schizophrenia, bipolar disorder (BP), delayed dyskinesia, and the like),
inflammatory reaction to oxidative stress,
Down syndrome (DS),
cancer,
cardiovascular diseases (arterial sclerosis (atherosclerosis and the like), ischemic cardiac diseases (cardiac angina, myocardial infarction and the like), heart failure, brain stroke, brain damage (for example, ischemic brain damage), reperfusion injury, hypertension, hyperhomocysteinemia and the like),
respiratory diseases (lung emphysema, bronchial asthma and the like),
lifestyle-related diseases (metabolic syndrome, diabetes, hypertension and the like),
skin disorders (dermatitis, photoaging of skin and the like),
gastrointestinal diseases (reflux esophagitis, gastric ulcer, inflammatory bowel disease and the like),
liver diseases (alcoholic liver disease, nonalcoholic liver disease and the like),
kidney diseases (kidney failure, glomerulonephritis and the like),
autoimmune diseases (connective tissue disease, articular rheumatism and the like),
otorhinolaryngologic diseases (mouth inflammation, hay fever and the like),
sepsis,
stress after organ transplantation,
chronic fatigue syndrome, and
age-related disease (physiological aging).

In an aspect of the present invention, the oxidative stress-related disorder is an eye disease, for example, retinal degeneration (age-related macular degeneration (AMD) (for example, age-related maculopathy based on oxidative stress), diabetic retinopathy or the like), cataract, or dry eye. Preferably, the oxidative stress-related disorder is age-related macular degeneration (AMD) (for example, age-related maculopathy based on oxidative stress). Reports have been presented on the role of oxidative stress in age-related macular degeneration (Beatty S et al. Survey Ophtalm. 2000; 45: pp. 115-134; (de Jong Paulus T V M Age-related macular degeneration. The New England journal of medicine, 2006; 355(14): pp. 1474-85); and Wu J, Seregard S et al. Survey Ophtalm. 2006; 51: pp. 461-481).

It has been reported that oxidative stress plays a major role in AMD lesion formation (Beatty S et al. Survey Ophtalm. 2000; 45: pp. 115-134). Increased levels of PUFA peroxidation products such as 4-hydroxynonenal (HNE) and 4-hydroxyhexanal (HHE) in the retina have been reported (Long EK et al. Free Rad. Biol. Med. 2010; 49: pp. 1-8). PUFA peroxidation products play a major role in formation of retinal pigment epithelium lipofuscin, and the lipofuscin itself may generate ROS during irradiation with a visible light ray, and plays a major role in pathogenesis of AMD (Katz ML, Arch. Gerontol. Geriatr. 2002; 34: pp. 359-370). A PUFA peroxidation product containing malondialdehyde (MDA) plays a noticeable role in lenticular pathology including formation of cataract as peroxidation of PUFA has been publicly reported to be an initiation step in human cataract lesion formation (Borchman D. et al. J. Lipid Res. 2010; 51: pp. 2473-2488). The role of PUFA peroxidation products in diseases of human corneas including pterygium and conical corneas is similarly important (Shoham A et al. FreeRad. Biol. Med. 2008; 45: pp. 1047 to 1055). The diabetic retinopathy is also related to oxidative stress and peroxidation of PUFA (Baynes JW, Thorpe SR. Diabetes, 1999; 48: pp. 1-9).

In an aspect of the present invention, the oxidative stress-related disorder is a neurological disease, for example, Alzheimer's disease, Parkinson's disease, synucleinopathy, mild cognitive impairment, amyotrophic lateral sclerosis (ALS), schizophrenia, bipolar disorder (BP) or the like. It has been reported that major neurological diseases are related to oxidative stress. For example, oxidated membrane components promote, through covalent and non-covalent mechanisms, β- and α-synuclein aggregations, which are related to Alzheimer's disease (AD) and Parkinson's disease (PD), and synucleinopathy. PUFA peroxidation reaction products can induce misfolding of protein in a sporadic amyloid disease which is the most clinically important neurological brain disease (Bieschke J. et al. ACC. Chem. Res. 2006; 39: pp. 611-619).

Reports on Alzheimer's disease (AD) and mild cognitive impairment (MCI) are presented in Cooper JL. Drugs & Aging, 2003; 20: pp. 399-418. Amyloid plaque and neurofibrillary tangles are neuropathological characteristics of AD, and there still is a room to discuss whether they are a cause or result of the disease. The oxidative stress and related inflammation are involved in the AD process. Direct evidences supporting increased oxidative stress in AD are studies indicating (1) an increase in amount of ROS-stimulating Fe, Al and Hg in the AD brain, (2) increased peroxidation of PUFA and a decrease in amount of PUFA in the AD brain, and an increase in amount of 4-HNE of AD cerebroventricular fluid, (3) increased oxidation of protein and DNA in the AD brain, (4) declined energy metabolism in the AD brain and a decrease in amount of cytochrome c oxidase, (5) advanced glycation end products (AGE), MDA, carbonyl, peroxynitrite, heme oxygenase-1 and SOD-1 in the neurofibrillary tangle, and AGE, heme oxygenase-1 and SOD-1 in the senile plaque, and (6) the fact that the amyloid β peptide can generate ROS (Markesbery WR. Free Rad. Biol. Med. 1997; 23: pp. 134-147).

The amyotrophic lateral sclerosis (ALS) is a delayed progressive neurodegenerative disease having influences on motor nerve cells (loss of upper and lower motor nerve cells), and results in death from muscle wasting and respiratory failure (Boillee S et al. Neuron 2006; 52: pp. 39-59). The pathogenesis for most ALS cases is still unknown, but ALS is recognized to be strongly related to oxidative stress. Familial ALS (fALS) is caused by oxidation of variant SOD (superoxide dismutase) (Kabashi E. et al., Ann. Neurol. 2007; 62: pp. 553-559). There are more than 100 variations of SOD which are related to fALS (Barnham KJ et al, Nature Rev. Drug Discov. 2004; 3: pp. 205-214). The first step is "monomerization" of SOD. Subsequently, SOD monomers aggregate, and abnormal S-S bonds are formed between the monomers (Kabashi E. et al, Ann. Neurol. 2007; 62: pp. 553-559), followed by formation of a toxic aggregate (Barnham KJ et al, Nature Rev. Drug Discov. 2004; 3: pp. 205-214). The theoretical pathogenesis for ALS cases is still unknown, but ALS is recognized to be related to oxidative stress and inflammation. In a certain test, oxidation of protein covers as high as 85% of sporadic ALS (sALS) patients (Coyle JT. et al, Science 1993; 262: pp. 689-695). An increase in both peroxidation of fat and formation of HNE in central nerve system (CNS) tissues, spinal fluid and serum has been reported for both familial and sporadic ALS cases (Simpson EP et al, Neurology 2004; 62: pp. 1758-1765). The source of oxidative stress in ALS has not been known, but can be generated from excitotoxity, mitochondrial dysfunction, iron accumulation or immune activation (Simpson EP et al, Neurology 2004; 62: pp. 1758-1765). There is an evidence that mitochondria play an important role in fALS and sALS, both of which are a cause and a target of oxidative stress in ALS (Bacman SR et al, Molec. Neurobiol. 2006; 33: pp. 113-131). Inhibition of cyclooxygenase 2 (COX-2) has been reported to reduce the spinal neurodegeneration, and extend the life of mice transgenic for an ALS gene (Minghetti L. J Neuropathol Exp Neurol 2004; 63: pp. 901-910), and this emphasizes the role of PUFA oxidation products in the pathogenesis of ALS. There is also an evidence that the HHE-protein conjugate in ALS patients increases (Long EK, Picklo MJ. Free Rad. Biol. Med. 2010; 49: pp. 1-8). Although oxidative stress is related to ALS, the approach of antioxidant therapy has not reached success yet (Barber SC et al, Biochim. Biophys. Acta 2006; 1762: pp. 1051-1067).

Parkinson's disease (PD) is related to oxidative stress caused by ROS, and ROS contributes to cascades that cause degeneration of dopamine cells in PD. However, the oxidative stress is closely related to other components of diseases and degenerative processes such as mitochondrial dysfunction, excitotoxity, nitrogen monoxide toxicity and inflammation. Formation of a toxic lipid peroxide in cells is considered to be directly related to damage in nigral nerve cells via activation of cell cascades. Oxidative damage related to PD starts at the PUFA level, and then spreads to protein, and nuclear DNA and mtDNA (for example, in synuclein processing/Lewy body formation), and a toxic carbonyl product of oxidative damage such as HNE and MDA may further react with the protein, leading to impairment of cell viability. Nitrogen monoxide is known to react with a superoxide to form peroxynitrite, and ultimately a hydroxyl group. A change in protein decomposition is considered very important for and linked to dopaminergic cell death in PD. The oxidative stress may directly impair these processes, and products of oxidative damage such as HNE may damage 26S proteasome. HNE is considered to be directly related to PD lesion formation (Selley ML. Free Rad. Biol. Med. 1998; 25: pp. 169-174; and Zimniak P, Ageing Res. Rev. 2008; 7: pp. 281-300). Further, the impairment of the proteasome function causes generation of free radicals and oxidative stress (Jenner P. Annals Neurol. 2003; 53: pp. S26-S36). An additional source of ROS related to the pathogenesis of PD is a metabolic turnover of dopamine (DA) in dopaminergic nerve cells (Hastings TG, J. Bioenerg. Biomembr. 2009; 41: pp.469-72). The oxidative damage to nucleic acids, which is mediated via the PUFA peroxidation product also contributes to the pathogenesis of PD (Martin LJ, J. Neuropathol. Exp. Neurol. 2008; 67: pp. 377-87; and Nakabeppu Y. et al, J. Neurosci. Res, 2007; 85: pp. 919-34). Regardless of whether or not the oxidative stress is cause or a result of PD, reduced oxidative stress is likely to have influences on the progress of disease.

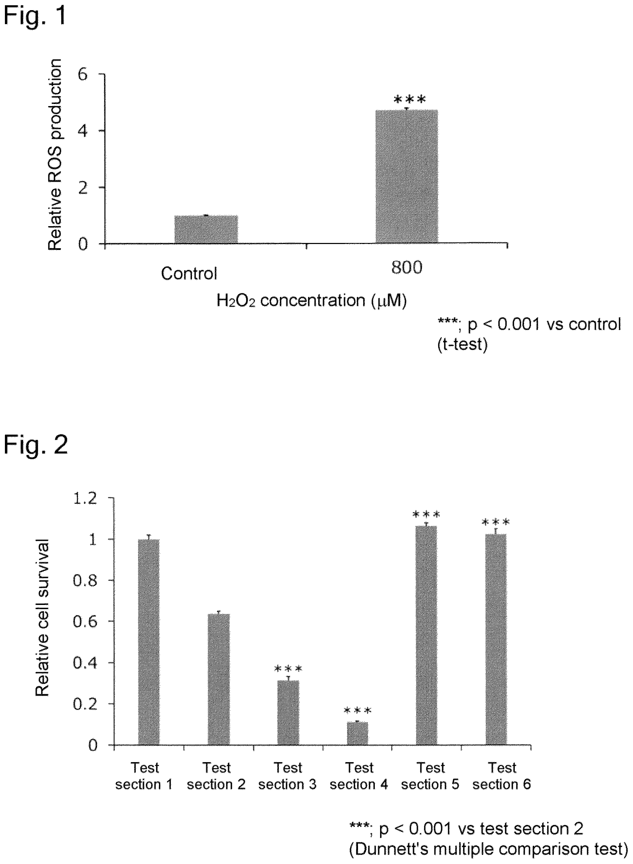

In both schizophrenia, bipolar disorder (BD), oxidative stress and the HNE level significantly increase (Wang JF et al, Bipolar Disorders 2009; 11: pp. 523-529). The impairment of the synaptic function is known to be an early pathological event in the neuropathology of AD, ALS, PD or the like (LoPachin RM et al., Neurotoxicol. 2008; 29: pp. 871-882). The molecular mechanism of the synaptic toxicity is unknown, but published evidences suggest that these diseases are characterized by a common pathophysiological cascade pertaining to oxidative stress, peroxidation of PUFA (Fig. 1), and subsequent release of α,β-unsaturated carbonyl derivatives such as acrolein and 4-HNE.

In an aspect of the present invention, the oxidative stress-related disorder is Down syndrome (DS). DS (trisomy of chromosome 21) is related to premature aging and intellectual disability which are similar to Alzheimer's disease. DS is associated with a high rate of incidence of autoimmune diseases and cataract, and this indicates increased oxidative stress in subjects of DS (Jovanovic SV et al, Free Rad. Biol. Med. 1998; 25: pp. 1044-1048). The chromosome 21 encodes Cu/Zn SOD and amyloid β-peptides, and therefore, DS is characterized by the overflow of the resulting gene products and metabolism products, in particular, a rise in ratio of SOD to catalase, which is associated with excess H2O2 (Sinet PM. Ann. NY Acad. Sci. 1982; 396: pp. 83-94). In the subject of DS, the amounts of markers of oxidation of protein and lipid (MDA, HNE and the like), and the amounts of ultimate glycosylation products and ultimate products of lipoxidation significantly increase (Busciglio J, Yankner BA. Nature 1995; 378: pp. 776-779; and Odetti P et al, Biochem. Biophys. Res. Comm. 1998; 243: pp. 849-851).

In an aspect of the present invention, the composition according to the present invention may be used for treating (healing or ameliorating) an oxidative stress-related disorder which has been already developed, or for preventing the onset of a predicted oxidative stress-related disorder.

In an aspect of the present invention, the composition according to the present invention may be used in combination with another therapeutic drug. In this case, the composition may be used for preventing or treating a side effect caused by the other therapeutic drug. Specific examples of the other therapeutic drug include anticancer agents, and antipyretic and pain relief drugs.

Examples of the anticancer agent include doxorubicin, cisplatin, vinblastine, mitomycin C, and camptothecin, and examples of the side effect thereof include fatigue, nausea, decreased appetite, depressive symptoms, bone-marrow suppression (leukocytopenia, amnesia, thrombocytopenia and the like), gastrointestinal tract disorders (mouth inflammation, diarrheal and the like), skin disorders (hair loss, nail change and the like), myocardial disorders, kidney disorders, lung disorders, and peripheral nerve disorders.

Examples of the antipyretic and pain relief drug include diclofenac sodium, loxofenac sodium, acetaminophen, and aspirin, and examples of the side effect thereof include liver disorders, kidney disorders, gastrointestinal tract disorders (erosive gastritis, gastric ulcer, duodenal ulcer and the like), and Stevens-Jonson syndrome.

In an aspect of the present invention, the composition according to the present invention may be used as a side effect prevention or treatment medical drug intended to prevent a side effect resulting from oxidative stress caused by another therapeutic drug.

In an aspect of the present invention, the composition according to the present invention may contain one or more pharmaceutically acceptable additives in addition to an active ingredient. The additive is not particularly limited as long as it is used as an additive for pharmaceutical products, and examples thereof include diluents (for example, solvents and excipients), binders, lubricating agents, glidants, plasticizers, disintegrants, buffers, and stabilizers.

In an aspect of the present invention, the dosage form of the composition according to the present invention can be appropriately selected according to a route of administration. In an embodiment, the composition is a solution or a soft capsule. The mode of administration may be oral administration or parenteral administration (for example, intravenous administration, intramuscular administration, subcutaneous administration, transdermal administration, intraspinal administration or intraocular administration), and is preferably oral administration. Preferably, the administration is performed every day for a plurality of days. For example, for obtaining a better antioxidation inducing action, the administration can be performed every day for 7 days or more, preferably 14 days or more.

In an aspect of the present invention, the composition according to the present invention can be used for alleviating oxidative stress. In an embodiment of the present invention, the composition can be used as a food composition (antioxidation inducing food composition) or a cosmetic composition (antioxidation inducing cosmetic composition). The composition for use in alleviation of oxidative stress can be used for alleviating, for example, muscular fatigue, mental fatigue, damaged skin and aging.

In an aspect of the present invention, the composition for use in alleviation of oxidative stress may contain one or more components acceptable as food in addition to an active ingredient. Examples of the component include flavors, sweeteners and colorants as well as the additives described above. More specifically, the food composition may be a nutritional supplementary food in the form of a conventional food composition such as a beverage, a confection, a dairy product, seasoning or a supplement.

In an aspect of the present invention, when the composition is in the form of food, the food is not particularly limited, and may be a beverage, confectionery, bread or soup. Examples thereof include common boil-in-the-bag foods, frozen foods, ready-to-eat foods (noodles and the like), canned foods, sausages, cookies, biscuits, cereal bars, crackers, snacks (potato chips and the like), pastries, cakes, pies, candies, chewing gum (including pellets and sticks), jellies, soups, ices, dressings, yogurts, supplements in the form of a tablet, a capsule and an emulsion, and soft drinks. The food is not particularly limited as long as it does not impair the effects of the present invention, which can be produced by a method used for producing the relevant food by a person skilled in the art.

In an aspect of the present invention, the composition for use in alleviation of oxidative stress may contain one or more components acceptable as a cosmetic in addition to an active ingredient. Examples of the component include solvents, surfactants, lubricants, stabilizers, colorants, preservatives. More specifically, the cosmetic composition may be a conventional quasi-pharmaceutical cosmetic product such as a cosmetic for skin or a cosmetic for hair.

In an aspect of the present invention, the composition for use in alleviation of oxidative stress may be an emulsified composition obtained by mixing an active ingredient as an oil phase with an aqueous phase. The emulsified composition may be an oil-in-water (O/W) emulsion composition obtained by adding an oil phase to a D phase (surfactant phase) containing a surfactant (nonionic surfactant or the like), water and glycerin, followed by addition of an aqueous phase.

In an aspect of the present invention, the implementation of the present invention includes selling a product according to the present invention with a packaging container (box, bag, can, bottle or the like), written instructions of the product or a pamphlet on which effects exhibited by the composition are displayed. The implementation of the present invention also includes displaying the effects of the present invention on televisions, internet websites, pamphlets, newspapers, magazines or the like to advertise and sell a product according to the present invention.

In an aspect of the present invention, the amount of an active ingredient selected from a very-long-chain polyunsaturated fatty acid, a pharmaceutically functional derivative thereof and a pharmaceutically acceptable salt thereof and taken by a subject is not particularly limited, and is, for example, equal to or more than an effective amount for obtaining a desired oxidative stress alleviating effect. In an aspect of the present invention, the amount of a component selected from a very-long-chain polyunsaturated fatty acid, a salt thereof and an ester thereof and taken by a subject is not particularly limited, and is, for example, equal to or more than an effective amount for obtaining a desired oxidative stress alleviating effect. For example, an adult may take an active ingredient at, in terms of a very-long-chain polyunsaturated fatty acid, 2 mg or more/kg of body weight/day, for example, 3 mg or more/kg of body weight/day, 4 mg or more/kg of body weight/day, 5 mg or more/kg of body weight/day, 6 mg or more/kg of body weight/day, 7 mg or more/kg of body weight/day, 8 mg or more/kg of body weight/day, 9 mg or more/kg of body weight/day, 10 mg or more/kg of body weight/day, 11 mg or more/kg of body weight/day, 12 mg or more/kg of body weight/day, 13 mg or more/kg of body weight/day, 14 mg or more/kg of body weight/day, 15 mg or more/kg of body weight/day, 16 mg or more/kg of body weight/day, 17 mg or more/kg of body weight/day, 18 mg or more/kg of body weight/day, 19 mg or more/kg of body weight/day, 20 mg or more/kg of body weight/day, 21 mg or more/kg of body weight/day, 22 mg or more/kg of body weight/day, 23 mg or more/kg of body weight/day, 24 mg or more/kg of body weight/day, 25 mg or more/kg of body weight/day, 30 mg or more/kg of body weight/day, 40 mg or more/kg of body weight/day, 50 mg or more/kg of body weight/day, 100 mg or more/kg of body weight/day or 200 mg or more/kg of body weight/day for 4 weeks or more, for example, 5 weeks or more, 6 weeks or more, 7 weeks or more, 8 weeks or more, 9 weeks or more, 10 weeks or more, 11 weeks or more or 12 weeks or more depending on conditions such as an age, a body weight and a health state of a subject.

An active ingredient selected from a very-long-chain polyunsaturated fatty acid, a pharmaceutically functional derivative thereof and a pharmaceutically acceptable salt thereof does not have strong side effects, and therefore there is no limitation on the amount of intake of the active ingredient per day. Similarly, a component selected from a very-long-chain polyunsaturated fatty acid, a salt thereof and an ester thereof does not have strong side effects, and therefore there is no limitation on the amount of intake of the component per day.

Examples of the present invention will be described below, but the present invention is in no way limited thereto.

### [Example 1] Evaluation of oxidative stress by quantifying amount of reactive oxygen species

Human alveolar epithelial cells were seeded on a 96-well microplate at 1.5 × 10⁴/well, and cultured overnight at 37°C and 5% CO₂. The medium was removed by suction, and exchanged for a medium containing 40 µM DCFH-DA (Wako Pure Chemical Industries, Ltd.), followed by incubation for 30 minutes. The medium was removed by suction, and washing was performed with PBS, followed by treatment with 800 µM hydrogen peroxide for 1 hour. The fluorescence intensity at each of 485 nm in excitation and 538 nm in emission was measured with a fluorescence plate reader. The relative fluorescence intensity of a treatment section was calculated, where the fluorescence intensity value of a well without hydrogen peroxide treatment was 1.

The results are shown in Fig. 1. The vertical axis of the graph represents the relative production amount of ROS (reactive oxygen species). The production amount of ROS in a section treated with 800 µM hydrogen peroxide is significantly larger than that in a non-treatment section.

### [Example 2] Evaluation of effect of protection from oxidative stress

Human alveolar epithelial cells were seeded on a 96-well microplate at 8 × 10³/well, and cultured overnight at 37°C and 5% CO₂. The medium was removed by suction, and exchanged for a medium containing a fatty acid ethyl ester (produced by a method described in US 2013/0190399A1), followed by incubation at 37°C and 5% CO₂ for 24 hours. The medium was removed by suction, and then exchanged for a medium containing 800 µM hydrogen peroxide, followed by incubation at 37°C and 5% CO₂ for 8 hours. CCK8 (DOJINDO LABORATORIES) was added to each well, and the absorbance of the sample at 450 nm after 1.5 hours was measured. The relative survival rate of each treatment section was calculated, where the absorbance value of a well without hydrogen peroxide treatment was 1.
Test section 1: control
Test section 2: hydrogen peroxide
Test section 3: 10 µM arachidonic acid (C20:4n-6) ethyl ester + hydrogen peroxide
Test section 4: 30 µM arachidonic acid (C20:4n-6) ethyl ester + hydrogen peroxide
Test section 5: 10 µM C32:4n-6 ethyl ester + hydrogen peroxide
Test section 6: 30 µM C32:4n-6 ethyl ester + hydrogen peroxide

The results are shown in Fig. 2. Fig. 2 is a graph showing relative cell survival rates after hydrogen peroxide treatment in human retinal pigment epithelial cells which were obtained in the test. The C32:4n-6 ethyl ester gave significantly higher cell survival rate over the arachidonic acid (C20:4n-6) ethyl ester at the same treatment concentration.

The cells treated with arachidonic acid (C20:4n-6) ethyl ester gave a significantly lower cell survival rate and the C32:4n-6 ethyl ester gave a significantly higher cell survival rate as compared to the section treated with hydrogen peroxide alone.

### [Example 3] Evaluation of effect of protection from oxidative stress

Human retinal pigment epithelial cells were seeded on a 96-well microplate at 1 × 10⁴/well, and cultured overnight at 37°C and 5% CO2. The medium was removed by suction, and exchanged for a medium containing a fatty acid ethyl ester of each concentration, followed by incubation at 37°C and 5% CO₂ for 24 hours. The medium was removed by suction, and then exchanged for a medium containing 300 µM hydrogen peroxide, followed by incubation at 37°C and 5% CO₂ for 24 hours. CCK8 (DOJINDO LABORATORIES) was added to each well, and the absorbance of the sample at 450 nm after 1.5 hours was measured. The relative survival rate of each treatment section was calculated, where the absorbance value of a well without hydrogen peroxide treatment was 1.
Test section 1: control
Test section 2: hydrogen peroxide
Test section 3: 10 µM arachidonic acid (C20:4n-6) ethyl ester + hydrogen peroxide
Test section 4: 30 µM arachidonic acid (C20:4n-6) ethyl ester + hydrogen peroxide
Test section 5: 10 µM C32:4n-6 ethyl ester + hydrogen peroxide
Test section 6: 30 µM C32:4n-6 ethyl ester + hydrogen peroxide

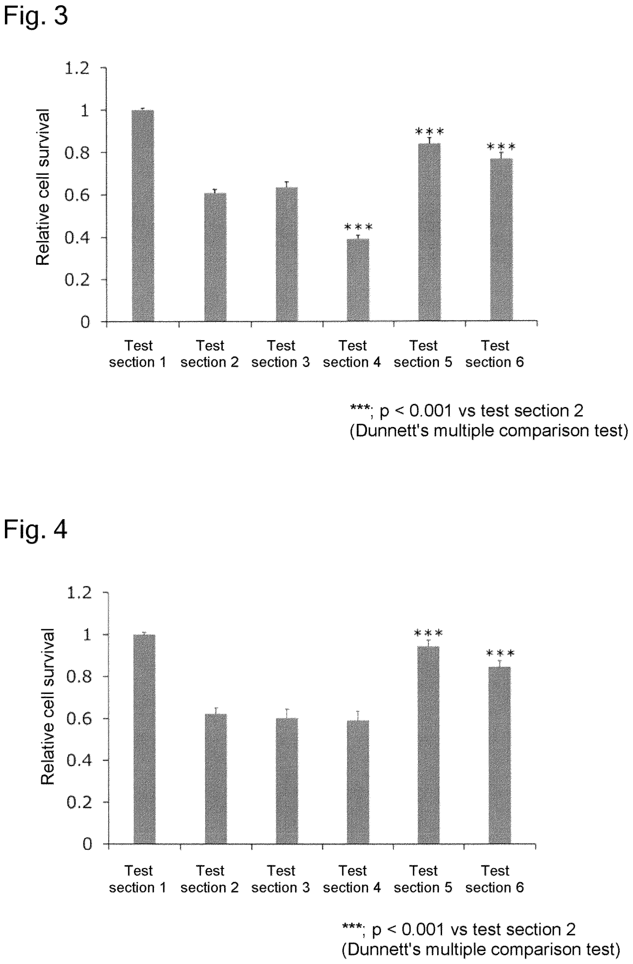

The results are shown in Fig. 3. Fig. 3 is a graph showing relative cell survival rates after hydrogen peroxide treatment in human retinal pigment epithelial cells which were obtained in the test. The C32:4n-6 ethyl ester gave a significantly higher cell survival rate over the non-treatment condition or the arachidonic acid (C20:4n-6) ethyl ester at the same treatment concentration.

### [Example 4] Evaluation of effect of protection from oxidative stress

Human retinal pigment epithelial cells were seeded on a 96-well microplate at 1 × 10⁴/well, and cultured overnight at 37°C and 5% CO₂. The medium was removed by suction, and exchanged for a medium containing a fatty acid ethyl ester of each concentration, followed by incubation at 37°C and 5% CO₂ for 24 hours. The medium was removed by suction, and then exchanged for a medium containing 300 µM hydrogen peroxide, followed by incubation at 37°C and 5% CO₂ for 24 hours. CCK8 (DOJINDO LABORATORIES) was added to each well, and the absorbance of the sample at 450 nm after 1.5 hours was measured. The relative survival rate of each treatment section was calculated, where the absorbance value of a well without hydrogen peroxide treatment was 1.
Test section 1: control
Test section 2: hydrogen peroxide
Test section 3: 10 µM eicosapentaenoic acid (C20:5n-6) ethyl ester + hydrogen peroxide
Test section 4: 30 µM eicosapentaenoic acid (C20:5n-6) ethyl ester + hydrogen peroxide Test section 5: 10 µM C34:5n-3 ethyl ester + hydrogen peroxide
Test section 6: 30 µM C34:5n-3 ethyl ester + hydrogen peroxide

The results are shown in Fig. 4. Fig. 1 is a graph showing relative cell survival rates after hydrogen peroxide treatment in human retinal pigment epithelial cells which were obtained in the test. The C34:5n-3 ethyl ester gave a significantly higher cell survival rate over the non-treatment condition or the eicosapentaenoic acid (C20:5n-3) ethyl ester at the same treatment concentration.

### INDUSTRIAL APPLICABILITY

The present invention provides a method for alleviating oxidative stress or a method for treating or preventing an oxidative-related disorder.

## Claims

1. A method for treating or preventing an oxidative stress-related disorder, the method comprising administering an effective amount of a very-long-chain polyunsaturated fatty acid (VLC-PUFA), a pharmaceutically functional derivative thereof or a pharmaceutically acceptable salt thereof to a subject in need of the treatment or prevention.

2. The method according to claim 1, wherein the pharmaceutically functional derivative is selected from the group consisting of an ester, an ether and an amide.

3. The method according to claim 1 or 2, wherein the pharmaceutically acceptable salt is selected from the group consisting of an inorganic salt or an organic salt.

4. The method according to any one of claims 1 to 3, wherein the very-long-chain polyunsaturated fatty acid is a fatty acid having 24 or more, 26 or more, 28 or more or 30 or more and 42 or less, 40 or less or 38 or less carbon atoms, containing 3 or more or 4 or more and 6 or less double bonds, and containing 0 or more and 1 or less, 2 or less or 3 or less hydroxy groups.

5. The method according to any one of claims 1 to 4, wherein the very-long-chain polyunsaturated fatty acid is a fatty acid represented by one of the following formulae: 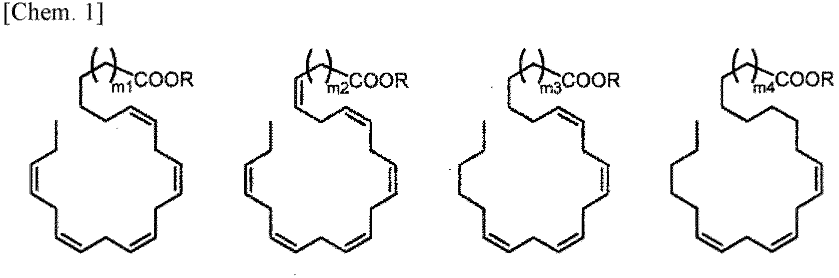 wherein m1, m2, m3 and m4 are each independently an integer selected from 4 to 22, 4 to 20, 6 to 20, 8 to 20, 10 to 20, 4 to 18, 6 to 18, 8 to 18 or 10 to 18, and R is a hydrogen atom.

6. The method according to any one of claims 1 to 5, wherein the very-long-chain polyunsaturated fatty acid is a very-long-chain polyunsaturated fatty acid selected from C32:4n-6, C34:4n-6, C32:5n-3, C34:5n-3, C32:6n-3, C34:6n-3, C32:3n-6 and C34:3n-6.

7. The method according to any one of claims 1 to 6, wherein the very-long-chain polyunsaturated fatty acid is a very-long-chain polysaturated fatty acid of C32:4n-6 or C34:5n-3.

8. The method according to any one of claims 1 to 7, wherein the very-long-chain polyunsaturated fatty acid (VLC-PUFA), pharmaceutically functional derivative thereof or pharmaceutically acceptable salt thereof is an ethyl ester of a very-long-chain polyunsaturated fatty acid.

9. The method according to any one of claims 1 to 8, wherein the oxidative stress-related disorder is selected from the group consisting of an eye disease, a neurological disease, an inflammatory reaction to oxidative stress, Down syndrome (DS), cancer, a cardiovascular disease, a respiratory disease, a lifestyle-related disease, a skin disorder, a gastrointestinal disease, a liver disease, a kidney disease, an autoimmune disease, an otorhinolaryngologic disease, sepsis, stress after organ transplantation, chronic fatigue syndrome and an age-related disease.

10. The method according to any one of claims 1 to 9, wherein the oxidative stress-related disorder is selected from the group consisting of age-related macular degeneration (AMD), cataract, diabetic retinopathy, Alzheimer's disease (AD), mild cognitive impairment (MCI), amyotrophic lateral sclerosis (ALS), Parkinson's disease (PD), Down syndrome (DS), schizophrenia, bipolar disorder (BD) and synucleinopathy.

11. The method according to any one of claims 1 to 10, wherein the oxidative stress-related disorder is selected from the group consisting of an inflammatory reaction to oxidative stress, retinal degeneration, atherosclerosis, amyotrophic lateral sclerosis (ALS), multiple sclerosis (MS), Friedreich ataxia, delayed dyskinesia, brain damage (for example, ischemia), photoaging of skin, reperfusion injury, brain stroke, myocardial infarction, hypertension, heart failure, epilepsy, hyperhomocysteinemia, physiological aging, sepsis and stress after organ transplantation.

12. The method according to any one of claims 1 to 11, wherein the oxidative stress-related disorder is a disorder caused by free radicals or reactive oxygen in a body.

13. The method according to any one of claims 1 to 12, wherein the oxidative stress-related disorder is a disorder related to the presence of ^{•}O₂⁻ (superoxide anion), H₂O₂ (hydrogen peroxide), *OH (hydroxyl radical), ROOH (organic hydroperoxide), RO* (alkoxyl radical), ROO' (peroxyl radical), HOCl (hypochlorous acid), OONO⁻ (peroxynitrite), NO', ¹O₂ (singlet oxygen), O₃ (ozone) or ^{•}NO₂ (nitrogen dioxide).
